# EUROPEAN PATENT SPECIFICATION

(11) **EP 2 247 284 B1**
(45) Date of publication and mention of the grant of the patent: **19.04.2017**
(21) Application number: 09713721.0
(22) Date of filing: 26.02.2009
(51) Int. Cl.: A61K 31/5517, A61K 47/42

(54) **PROCESS TO MINIMIZE POLYMORPHISM**
VERFAHREN ZUR MINIMIERUNG VON POLYMORPHISMUS
PROCÉDÉ VISANT À RÉDUIRE LE POLYMORPHISME

(30) Priority: 28.02.2008 US 32298 P
(43) Date of publication of application: 10.11.2010
(73) Proprietor: R.P. Scherer Technologies, LLC, Las Vegas, Nevada 89119 (US)
(72) Inventor: HOWES, Simon, A. M., Swindon Wiltshire SN2 2AR (GB); MCLAUGHLIN, Rosie, Swindon Wiltshire SN4 7JR (GB); JORDAN, Andrew, Swindon Wiltshire SN25 4TT (GB); TIAN, Wei, Minety Malmesbury SN16 9QS (GB)
(74) Representative: Dunleavy, Kevin James
(86) International application number: PCT/US2009/035276
(87) International publication number: WO 2009/108775

(56) References cited:
- WO-A1-03/000294
- DE-A1- 4 428 986
- US-A1- 2007 043 032
- US-B1- 6 509 040
- US-B1- 6 709 669
- US-B1- 6 709 669

## Description

### BACKGROUND OF THE INVENTION

### Field of the Invention

The instant invention relates to a process to minimize polymorphism by controlling crystallization, particularly to a method utilizing standard molecular weight (SMW) fish gelatin as a matrix former in a fast dispersing dosage form (FDDF), along with reduced dosing temperatures.

### DESCRIPTION OF RELATED ART

Drugs which form crystalline solids often exist in more than one crystal form, and each of these forms may have distinct properties in terms of solubility, melting point, bioavailability, etc. This capacity, deemed crystalline polymorphism, is of great concern to the pharmaceutical industry for its implications for causing variability in drug dosage forms. In a polymorphic substance, one of the crystal forms may be more stable or easier to handle than another, although the conditions under which the various crystal forms appears may be so close as to be very difficult to control on the large scale. Complex molecules used by the pharmaceutical industry tend to form polymorphs; these are typically distinguished by different molecular conformations in the crystal. X-ray diffraction is a standard method for determining crystal structures.
Polymorphism can create differences in the bioavailability of the drug which leads to inconsistencies in efficacy. In some cases, one crystal form can be spontaneously transformed into another during storage.

Polymorphism is often characterized as the ability of a drug substance to exist as two or more crystalline phases that have different arrangements and/or conformations of the molecules in the crystal lattice. Amorphous solids consist of disordered arrangements of molecules and do not possess a distinguishable crystal lattice. Solvates are crystalline solid adducts containing either stoichiometric or nonstoichiometric amounts of a solvent incorporated within the crystal structure. If the incorporated solvent is water, the solvates are also commonly known as hydrates. As defined in the International Conference on Harmonization (ICH) Guideline Q6A (2), the term polymorphism includes both solvate products and amorphous forms.

Polymorphs and/or solvates of a pharmaceutical solid can have different chemical and physical properties such as melting point, chemical reactivity, apparent solubility, dissolution rate, optical and electrical properties, vapor pressure, and density. These properties can have a direct impact on the processing characteristics of drug substances and the quality or performance of drug products, such as stability, dissolution, and bioavailability. A meta-stable pharmaceutical solid form can change crystalline structure or solvate or desolvate in response to changes in environmental conditions, processing, or even spontaneously over time.

Many drugs are intended to be administered in crystalline form, or crystallize partially, or completely, during manufacturing, handling, or storage. The Food and Drug Administration (FDA) approves many such drugs only in a specific crystal structure or polymorph. Different polymorphs have different solubility, different residence times in the body, and different therapeutic values. There are a number of examples in which polymorphic molecules change crystal structure under processing conditions while in contact with liquids or solid materials. In these environments, it is difficult to apply standard techniques to identify and predict the transformations. Furthermore, little is known about how to control polymorphic forms.

The FDA may refuse to approve an Abbreviated New Drug Application (ANDA) referencing a listed drug if the application contains insufficient information to show that the drug substance is the "same" as that of the reference listed drug. A drug substance in a generic drug product is generally considered to be the same as the drug substance in the reference listed drug if it meets the same standards for identity. In most cases, the standards for identity are described in the United States Pharmacopoeia (USP), although the FDA may prescribe additional standards when necessary. Because drug product performance depends on the product formulation, the drug substance in a proposed generic drug product need not necessarily have the same physical form (particle size, shape, or polymorph form) as the drug substance in the reference listed drug. An ANDA applicant is required to demonstrate that the proposed product meets the standards for identity, exhibits sufficient stability and is bioequivalent to the reference listed drug.

Since polymorphs exhibit certain differences in physical characteristics (for example, powder flow and compaction, apparent solubility and dissolution rate) and solid state chemistry (reactivity), attributes that relate to stability and bioavailability, it is essential that the product development and the FDA review process pay close attention to these issues. This scrutiny is essential to ensure that polymorphic differences (when present) are addressed via design and control of formulation and process conditions for physical and chemical stability of the product over the intended shelf-life, bioavailability and bioequivalence.

The solid state characteristics of drugs are known to potentially exert a significant influence on the solubility parameters. Polymorphs of a drug substance can have different apparent aqueous solubility and dissolution rate, when such differences are sufficiently large, the bioavailability is altered and it is often difficult to formulate a bioequivalent drug product using a different polymorph.

Solubility, at a defined temperature and pressure, is the saturation concentration of the dissolved drug in equilibrium with the solid drug. Aqueous solubility of drugs is traditionally determined using the equilibrium solubility method that involves suspending an excess amount of a solid drug in a selected aqueous medium. The equilibrium solubility method may not be suitable to determine the solubility of a meta-stable form, since the meta-stable form may convert to the stable form during the experiment.

Polymorphs of a pharmaceutical solid may have different physical and solid state chemical (reactivity) properties. The most stable polymorphic form of a drug substance is often used because it has the lowest potential for conversion from one polymorphic form to another, while the meta-stable form may be used to enhance the bioavailability. Gibbs free energy, thermodynamic activity, and solubility provide the definitive measures of relative polymorphic stability under defined conditions of temperature and pressure. The relative polymorphic stability may be determined by an iterative examination of the relative apparent solubility of supersaturated solutions of polymorphic pairs. Since the rate of conversion to the more stable form is often rapid when mediated by the solution phase, the less stable polymorph with the greater apparent solubility dissolves, while the more stable polymorph with the lower apparent solubility crystallizes out upon standing.

Solid-state reactions include solid-state phase transformations, dehydration and desolvation processes, and chemical reactions. One polymorph may convert to another polymorph during manufacturing and storage, particularly when a meta-stable form is used. Since an amorphous form is thermodynamically less stable than any crystalline form, inadvertent crystallization from an amorphous drug substance may occur. As a consequence of the higher mobility and ability to interact with moisture, amorphous drug substances are also more likely to undergo solid-state reactions.

In addition, phase conversions of some drug substances are possible when exposed to a range of manufacturing processes. Milling and/or micronization operations may result in polymorphic form conversion of a drug substance. In the case of wet granulation processes, where the usual solvents are aqueous, one may encounter a variety of inter-conversions between anhydrates and hydrates, or between different hydrates. Spray-drying processes have been shown to produce amorphous drug substances.

A typical drug exhibiting problems of polymorphism is alprazolam, a benzodiazepine. Alprazolam is indicated for the management of central nervous systems disorders such as anxiety disorder or the short-term relief of symptoms of anxiety. Alprazolam displays considerable polymorphic crystalline behavior when attempts are made to incorporate it in a FDDF. Alprazolam is poorly soluble in water. It undergoes polymorphism upon exposure to an aqueous environment, with as many as five crystal modifications forming. The industrial manufacture of a FDDF may require holding the drug suspension in water for up to 48 hours. The changes in crystal sizes and morphology that can occur over this 48 hour period may lead to severe difficulty in the uniform dosing of the drug suspension throughout the batch. The crystal modifications at different time points in the dosing period may lead to significant differences in crystal morphology of the finished dosage forms.

Previous approaches have included attempts to create more stable crystalline forms of alprazolam. For example, German patent DE 289468 A5 discloses a method of manufacturing an alprazolam dosage form with good bioavailability and uniformity. The manufacturing method comprises the steps of: 1) converting the alprazolam by hydration into a fine crystalline dihydrate; 2) adding a viscosity increasing agent and 3) applying the suspension to a solid galenic form. The preferred hydration step includes the suspension of 1 part of alprazolam in about 10 to 30 parts, w/w, of water. Preferred viscosity increasing agents include sodium carboxymethylcellulose (2%) and gelatin (10%).
DE 4428986 A1 also relates to solid alprazolam formulations which include a carrier comprising 1-70 wt% water,-soluble α,β or ψ-cyclodextrin, mono-, di-, oligo- or polysaccharide or polyvinylpyrrolidone of average molecular weight of 25000-120000; (b) 0-10 wt% water-insoluble hydrophilic porous or non-porous SiO₂, TiO₂ or Al₂O₃; and/or (c) 0-10 wt% non-ionic surfactant having an HLB value of 13-18; and (ii) 2-20 wt% disintegrant comprising sodium starch gycolate, crosslinked polyvinylpyrrolidone or sodium carboxymethyl cellulose. This composition is said to reduce polymorphism of amorphous alprazolam.

This approach of seeking more stable crystalline forms as a potential solution to polymorphism is widespread. In the instant invention, a very different approach has been found to be successful. Instead of attempting to promote more stable crystalline forms, the instant invention tends to suppress the conversion to new crystalline structures.

The FDDF is a well-known dosage form. Many references describe them as "fast disintegrating", "fast dissolving", "fast dispersing", "rapidly disintegrating" and the like. These references disclose how to prepare the FDDF and how it measure the disintegration times. These references include
Patent Nos. 6,083,531; 5,958,453; 5,273,759; 5,457,895; 5,720,974; 5,869,098; 5,631,023; 6,010,719; 4,371,516; and 4,946,684.

A person who is under medical distress from the sudden attack of a condition such as anxiety, as well as panic disorder, could achieve rapid release of the active ingredients through the use of a FDDF. The FDDF will dissolve rapidly, without leaving any intractable, insoluble residue upon which the user might choke. In other applications the FDDF will also provide a convenient dosage form. Children, elderly, and other users often have difficulty swallowing pills or capsules, particularly without supplemental water to drink and the present invention overcomes these problems for active ingredients that are subject to polymorphism.

Experience has long shown that pharmaceuticals or other items for human or animal consumption may be safely packaged in a FDDF. Gelatin is a protein/food ingredient, obtained by the thermal denaturation of collagen, which is the most common structural material and most common protein in animals. Gelatin forms thermally reversible gels with water, which gives gelatin products unique properties, such as reversible sol-gel transition states at near physiologic temperatures. Thus, gelatin is a preferred structural former for FDDFs.

Gelatin is an amphoteric protein with an isoionic point between 5 and 9, depending on raw material and method of manufacture. Type A gelatin, with an isoionic point of 7 to 9, is derived from collagen with acid pretreatment. Type B gelatin, with an isoionic point of 4.8 to 5.2, is the result of alkaline pretreatment of the collagen. Like its parent protein collagen, gelatin is unique in that in contains, approximately, 16 % proline, 26 % glycine, and 18% nitrogen. Gelatin is not a complete protein food because the essential amino acid tryptophan is missing and the amino acid methionine is present only at a low level.

There are a large number of processes used in the manufacture of gelatin and the raw materials from which it is derived, including demineralized bone, pigskin, cow hide and fish. The proteinaceous material, collagen, and hence gelatin, can be derived from any edible protein containing material. For reasons of economy, gelatin can be most practically be derived from protein sources which would normally require refining before consumption and which would otherwise make up protein-containing waste material destined for animal feeds, agricultural fertilizers, or for other industries. However, in many cultures and areas of the world, gelatin processed from mammalian origins, that is, from beef or pigs, is not acceptable.

In the fish industry, there is considerable and unavoidable waste of fish protein, especially from the fish skins that remain after processing. The fish skin which remains after processing, especially filleting, is generally inedible as such, but can be used in the glue industry or for the manufacture of animal foodstuffs, fertilizers or other commodities of low commercial value.

However, fish skins have become a vital commercial source of gelatin. In general, the fish collagen is acidified to about pH 4 and then heated stepwise from 50° C to boiling to denature and solubilize the collagen. Then, the denatured collagen or gelatin solution has to be defatted, filtered to high clarity, concentrated by vacuum evaporation or membrane ultra- filtration treatment to a fairly high concentration for gelation, and dried by passing dry air over the gel. Finally, the dried gelatin is ground and processed into powder. The resulting gelatin has an isoionic point of 7 to 9 based on the severity and duration of the acid processing of the collagen which causes limited hydrolysis of the asparagine and glutamine amino acid side chains.

U.S. Patent Application Publication No. 2001/0024678 details the manufacture of hard capsules from fish gelatin by means of adding a setting system comprising a hydrocolloid or mixtures of hydrocolloids and cations which may contain additional sequestering agents. WO 03/000294 A1 describes a pharmaceutical composition comprises a dispersion comprising a low-solubility drug and a matrix combined with a concentration-enhancing polymer. At least a major portion of the drug is amorphous in the dispersion. The compositions improve the stability of the drug in the dispersion, and/or the concentration of drug in a use environment. U.S. Patent no. 6,709,669 B1 relates to a pharmaceutical composition comprising a carrier and an active ingredient, wherein the carrier is fish gelatin and the composition is a fast-dispersing dosage form designed to release the active ingredient rapidly on contact with a fluid. In one embodiment, the composition is designed for oral administration and releases the active ingredient rapidly in the oral cavity on contact with saliva. The fish gelatin can be obtained from cold water fish sources and is preferably the non-gelling, non-hydrolyzed form. A process for preparing such a composition and a method of using fish gelatin in a fast dispersing dosage form are also provided.

As used in this specification and in the claims the term "standard molecular weight" (SMW) fish gelatin means a fish gelatin that has a stable solution or suspension viscosity at sub-ambient temperatures. Further, at a temperature of 15°C or less, and at a concentration of 10% by weight or less, the viscosity of the dosing solution or suspension changes by less than 50% from the original viscosity during a 48 hour holding time. Another understanding of SMW fish gelatin includes fish gelatins in which at least 50%, preferably more than 60% and most preferably more than 70% by weight of the molecular weight distribution is below 30,000 Daltons. Representative SMW fish gelatins useful in
the present invention include those supplied by Norland Products, Inc. of Cranbury, New Jersey.

A wide variety of active substances that are prone to polymorphism and administered orally may benefit from the instant invention. These include but are not limited to acyclovir, alendronate sodium, amoxicillin, aripiprazole, atorvastatin calcium, carbamazepine, carvedilol, cephalexin, clindamycin, colchicine, donepezil hydrochloride, erythromycin, esomeprazole magnesium, fluoxetine hydrochloride, hydrochlorothiazide, hydrocodone, hyoscyamine sulphate, levofloxacin, levothyroxine sodium, lisinopril, losartan potassium, methotrexate, mirtazapine, mometasone furoate monohydrate, morphine, nystatin, pantoprazole sodium, paroxetine hydrochloride, risedronate sodium, rosiglitazone maleate, tetracycline, theophylline and zithromax.

### SUMMARY OF THE INVENTION

A new paradigm for minimizing crystalline polymorphism in pharmaceutical substances that are susceptible to polymorphism is described. The inventive process and its products are defined by the claims. During formulation, the pharmaceutical substances are suspended in an aqueous matrix comprising a standard molecular weight (SMW) fish gelatin at a temperature lower than that of conventional processing schemes, that is, less than about 15°C. The suspension is then held at about that temperature during processing, which may require holding times longer than 24 hours. During this time, the combination of the fish gelatin and the low handling temperatures appears to have a synergistic effect that tends to suppress crystal formation in benzodiazepines, particularly, alprazolam. The resulting dosage forms therefore have pharmaceutical agents with less crystal polymorphism than products produced with bovine gelatin, high molecular weight (HMW) fish gelatin or pullulan as matrix forming agents and less crystal polymorphism than products produced at higher temperatures, regardless of the matrix forming agent.

There is also disclosed a process for preparing an oral, solid FDDF of a pharmaceutically active substance comprising the steps of: (a) forming a suspension of particles of at least one species selected from the class of drugs comprising those susceptible to crystalline conversion when exposed to aqueous environment, in a continuous phase in a carrier material, wherein the carrier material further comprises standard molecular weight fish gelatin; (b) reducing the suspension temperature to less than about 15°C; (c) maintaining the suspension temperature at less than about 15°C; (d) forming discrete units of the suspension at a formation temperature of less than about 15°C; and (e) removing the continuous phase to leave the suspension of particles in the carrier material.

### BRIEF DESCRIPTION OF THE DRAWINGS

FIG. 1 is a photomicrograph taken of a suspension of alprazolam observed shortly after suspension, showing relatively few, small, crystals;
FIG. 2 is a photomicrograph taken of a suspension of alprazolam in water, with gelatin and mannitol, 48 hours after suspension, showing numerous small needle-shaped crystals; and
FIG. 3 is a photomicrograph taken of a suspension of alprazolam in water, without any matrix forming agent, 48 hours after suspension, showing numerous large, rhomboid-shaped crystals.

### DETAILED DESCRIPTION OF THE INVENTION

The process and FDDF of the instant invention provides a significant advancement in the state of the art. The preferred embodiments of the process are configured in unique and novel ways and demonstrate previously unavailable but preferred and desirable capabilities.

The detailed description set forth herein is intended merely as a description of the presently preferred embodiments of the invention and is not intended to represent the only form in which the present invention may be prepared or utilized. The description sets forth the designs, functions, means, and methods of implementing the invention in connection with the described embodiments.

As is well known in the art, the commercial dosing of many pharmaceutical and similar products requires prolonged holding periods wherein large batches of product are individually formed and packaged. This is particularly true with FDDFs, where holding times of 24 hours or more are possible. During this holding period, many formulations are susceptible to the problems of polymorphic crystallization.

One such product is alprazolam, commonly known and manufactured under the trade name XANAX™ by Pfizer Corporation of New York, NY. Alprazolam is a triazole analog of the 1,4 benzodiazepine class of central nervous system- active compounds. The chemical name of alprazolam is 8-chloro-1- methyl-6-phenyl-4H-s-triazolo [4,3-α] [1,4] benzodiazepine.

Alprazolam is a white, crystalline powder, which is soluble in methanol or ethanol but which has no appreciable solubility in water at physiological pH. Central Nervous System (CNS) agents of the 1,4 benzodiazepine class presumably exert their effects by binding at stereo specific receptors at several sites within the central nervous system. Clinically, all benzodiazepines cause a dose-related central nervous system depressant activity varying from mild impairment of task performance to hypnosis. Alprazolam is indicated for the management of CNS disorders such as anxiety disorder or the short-term relief of symptoms of anxiety, as well as for the treatment of panic disorder.

The nature of the indications for alprazolam makes it an ideal candidate for the fast dispersing dosage form of administration. However, it was believed that the lengthy holding period involved in the commercial manufacture of a benzodiazepine FDDF might lead to unacceptable polymorphic crystallization of the drug during the dosing period.

Accordingly, a background study was performed to investigate the crystallizing behavior of alprazolam in FDDF formulations. In this experiment,
viscosity measurements were made using a Haake VT550 viscometer. Particle size was determined using a Malvern Mastersizer particle size analyzer, which measures the particle size of the test samples by laser diffraction. Purified water was used as the dispersant for all the samples tested and a sample obscuration of between 12% and 20% was achieved for each measurement. Each sample was measured three times and a mean d90 value calculated. The d90 value represents the 90^{th} percentile of particle size (i.e., 90% of all particles in the sample are of a lesser size than the d90 value).

The background study was undertaken to determine the magnitude of the polymorphic crystallization problem with prolonged holding times of alprazolam suspensions, using both a suspension of alprazolam in water and a test alprazolam suspension in gelatin and mannitol. Both suspensions, detailed in Table 1 below, were allowed to stand at ambient temperature (approximately 23°C) for 48 hours and then microscopically examined for crystal structure.

**Table 1: Alprazolam Solutions in Gelatin/Mannitol and Water**

| Experiment Number | 1 (% w/w) | 2 (% w/w) |
|---|---|---|
| Purified Water EP/USP | 92.20 | 99.20 |
| Alprazolam EP | 0.80 | 0.80 |
| Standard Molecular Weight Fish Gelatin EP/USP/JP | 4.00 | |
| Mannitol EP/USP | 3.00 | |
| TOTAL | 100.00 | 100.00 |

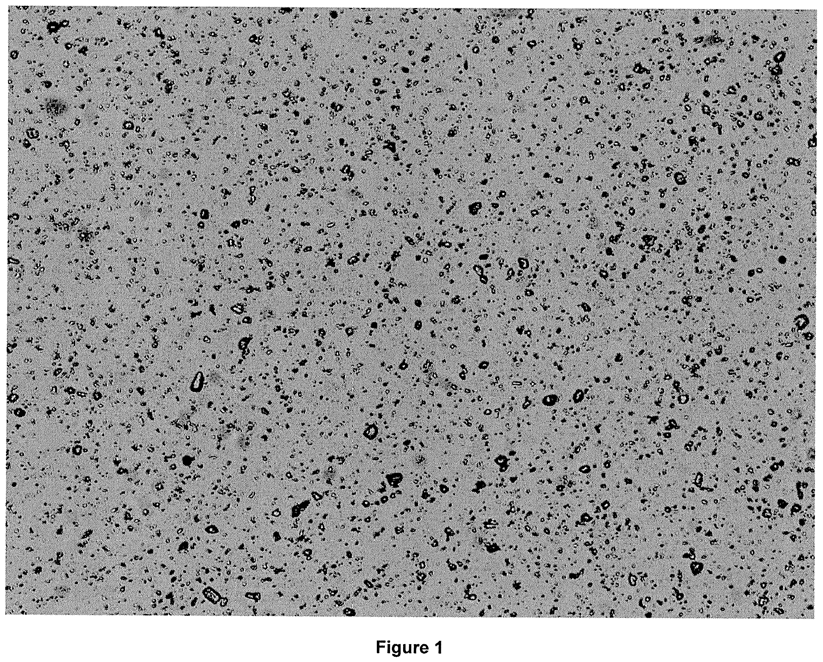

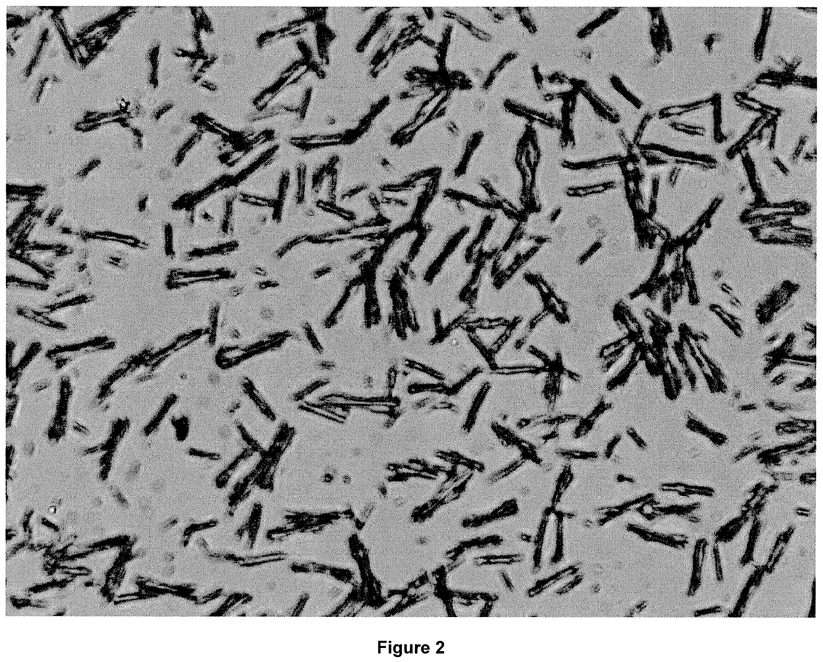

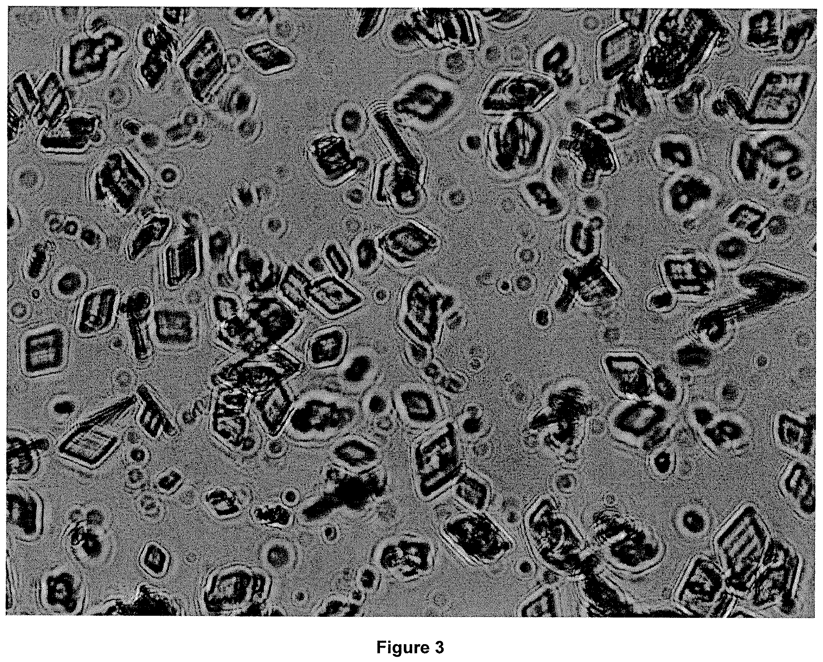

Microscopic evaluation of the initial suspensions at time zero showed relatively few, small, crystals, as seen in FIG. 1. After 48 hours, microscopic evaluation of batch Exp. 1, in which alprazolam was suspended (in water) with gelatin and mannitol, showed numerous small needle-shaped dihydrate crystals, as seen in FIG. 2. In contrast, microscopic evaluation of Exp. 2, containing only alprazolam and water, without any matrix forming agent, showed numerous large, rhomboid-shaped monohydrate crystals, as seen in FIG. 3. These polymorphic changes can be both problematic for the processing of the FDDF formulation and detrimental to drug bioavailability, as discussed previously.

Gelatins may affect the formation of crystals in gelatin solution. Therefore, experimentation was undertaken with alprazolam in various types of gelatin, to examine the formation of crystals in the resulting formulations. Gelatins employed included mammalian (bovine) gelatin (Gelatin EP/USNF), High Molecular Weight (HMW) Fish Gelatin, and Standard Molecular Weight (SMW) Fish Gelatin. Formulation details are given below in Table 2.

**Table 2: Formulation Details for Suspensions of Alprazolam in Various Gelatin Types**

| Exp. Number | 3 | 4 | 5 | 6 |
|---|---|---|---|---|
| Material | % w/w | % w/w | % w/w | % w/w |
| Purified Water | 92.2000 | 92.2000 | 92.2000 | 92.2000 |
| Alprazolam EP | 0.8000 | 0.8000 | 0.8000 | 0.8000 |
| Gelatin EP/USNF | 4.0000 | | | |
| Gelatin (GDF Source) | | 4.0000 | | |
| Gelatin EP/USP/JP (HMW Fish Gelatin) | | | 4.0000 | |
| Gelatin EP/USP/JP (SMW Fish Gelatin) | | | | 4.0000 |
| Mannitol EP/USP | 3.0000 | 3.0000 | 3.0000 | 3.0000 |

In addition, batches Exp. 5 (HMW Fish Gelatin) and Exp. 6 (SMW Fish Gelatin) were split into sub-batches in order to evaluate the effect of temperature. The Exp. 5 (HMW Fish Gelatin) batch was split into one batch maintained at 19°C (Exp. 5a) and one batch maintained at 23°C (Exp. 5b), while batch Exp. 6 (SMW Fish Gelatin) was divided into three batches: one maintained at 10°C (Exp. 6a), one maintained at 19°C (Exp. 6b) and one maintained at 23°C (Exp. 6c). Batches containing bovine gelatin only, that is, batches Exp. 3 and Exp. 4, were maintained at 23°C only.

The suspensions were maintained at the set temperatures for a holding period of 48 hours and samples were continuously stirred during that period. Samples were taken at various time points over the holding period and analyzed for signs of crystal change using light microscopy, particle size analysis, and viscosity testing. The microscopic examination results are seen in Table 3.

**Table 3: Observations of Crystal Formation of Alprazolam Solutions in Various Gelatin Matrix Systems and at Various Holding Temperatures**

| Exp. Number | Experiment Details | Results |
|---|---|---|
| Exp. 3 | Bovine Gelatin, 23°C | Extensive conversion. Mostly needle-shaped crystals after 18 hrs. |
| Exp. 4 | Bovine Gelatin, 23°C | Conversion to needle-shaped crystals evident after 6hrs, this increased in number after 18hrs. Full conversion after 48 hrs. |
| Exp. 5a | HMW Fish Gelatin, 19°C | Conversion to needle-shaped crystals evident after 6 hrs, this increased in number after 18hrs. Full conversion after 48 hrs. |
| Exp. 5b | HMW Fish Gelatin, 23°C | Needle-shaped crystals were seen immediately after mixing. Full conversion after 48 hrs. |
| Exp. 6a | SMW Fish Gelatin, 10°C | Conversion to needle-shaped crystals was seen after 18 hrs. Significant, but not complete conversion was seen after 48hrs. |
| Exp. 6b | SMW Fish Gelatin, 19°C | Needle-shaped crystals were seen immediately after mixing. Mostly needle shaped crystals after 18 hrs. |
| Exp. 6c | SMW Fish Gelatin, 23°C | Conversion to needle-shaped crystals evident after 6 hrs, this increased in number after 18 hrs. Full conversion after 48 hrs. |

The results from these batches (Table 3) show that a crystal conversion does take place in the alprazolam-gelatin suspension. The results also indicate that the temperature of the suspension is also having an effect, with batch Exp. 6a (SMW Fish Gelatin held at 10°C) not showing signs of conversion until 18 hours of stirring, compared to all other batches which showed signs of conversion after only 6 hours of stirring. The type of gelatin did not seem to be significant at higher temperatures, as all batches at 23°C showed signs of conversion after 6 hours stirring. Overall, batch Exp. 6a, alprazolam in SMW fish gelatin held at 10°C, showed the least crystal formation, and therefore was the pharmaceutically most advantageous.

A follow up experiment, in which the temperature of a SMW fish gelatin and alprazolam suspension was further decreased to 5°C, further confirmed both the stability of viscosity and particle size in several suspensions held at differing pH levels, as seen in Table 4.

**Table 4: Alprazolam Formulations in SMW Fish Gelatin at 5°C**

| Exp. Number | | Exp. 7 | Exp. 8 | Exp. 9 |
|---|---|---|---|---|
| Formulation Details Balance to water | | 4% SMW Fish Gelatin | 4% SMW Fish Gelatin | 4% SMW Fish Gelatin |
| | | 3% Mannitol Citric acid to pH 3.5 | 3% Mannitol Citric acid to pH 4.0 | 3% Mannitol Citric acid to pH 4.5 |
| | | 0.8% Alprazolam | 0.8% Alprazolam | 0.8% Alprazolam |
| Viscosity (mPas) | 0 hr | 7.27 | 6.41 | 6.03 |
| | 48 hr | 7.44 | 6.93 | 6.58 |
| Particle Size d90 (µm) | 0 hr | 12.85 | 12.45 | 13.99 |
| | 48 hr | 11.11 | 9.38 | 11.10 |

These experiments, however, while showing the efficacy of a SMW fish gelatin as a gel former in an alprazolam suspension held at 10°C or lower, did not eliminate the possibility that the effect observed was related, either primarily or significantly, to the temperature at which the suspension was held, rather than the gelatin itself. As bovine and HMW fish gelatins cannot be dosed at temperatures as low as 10°C, another series of experiments was undertaken to control for the effect of changing the gel forming matrix to a non-gelatin substance, while maintaining the low (10°C) temperature.

Accordingly, suspensions of alprazolam were made in SMW fish gelatin and pullulan and held at 10°C, to ascertain the effect of changing the matrix forming agent while maintaining drug type and concentrations steady. Results are seen in Table 5.

**Table 5: Comparison of Alprazolam Suspensions Utilizing Standard Molecular Weight Fish Gelatin and Pullulan at 10°C**

| Exp. Number | Exp. 10 | Exp. 11 |
|---|---|---|
| Formulation | 4% SMW Fish | 5% Pullulan |
| Details | Gelatin | 5% Mannitol |
| | 3% Mannitol | Citric Acid to pH 3 |
| Balance to water | Citric Acid to pH 3 | 0.8% Alprazolam |
| | 0.8% Alprazolam | |
| Viscosity mPas) | 7.07 | 27.23 |
| Particle Size d90 (µm) | 10.34 | 25.17 |
| Macroscopic Examination | Some small needle shaped crystals seen after 18 hours stirring | Some small needle shaped crystals at 18 hours stirring, but mainly rhomboid shaped crystals |

These results clearly indicated that pullulan was unable to sustain the inhibition of crystal formation, at the given temperatures, that was possible in the SMW fish gelatin formulation.

An initial extension of the experimentation was undertaken to evaluate suspensions of another benzodiazepine drug (Drug A). Drug A is a psychotropic agent that belongs to the thienobenzodiazepine class. It is clinically indicated for the treatment of schizophrenia and bipolar (manic-depressive) disorder. As a follow-up evaluation, formulations based on Standard Molecular Weight (SMW) fish gelatin were evaluated over extended time periods. A batch was manufactured at a 20 mg/unit strength, to study and attempt to confirm the effect of suspension temperature on the rate of the crystal conversion.

Exp. 12 was manufactured using Gelatin EP/USP/JP (SMW Fish Gelatin) as the matrix former. The batch was 400 g in size. The batch was held at 10°C for 72 hours and evaluated for signs of crystal conversion. The batch was assessed using viscosity, particle size and microscopic evaluation after 0, 24, 48, and 72 hours stirring. Dosing was also carried out at these time points. Samples were tested using a Haake VT 550 viscometer. The viscosity of the samples was determined at a shear rate of 2500 s⁻¹ at 10°C. Samples were also tested using a Malvern Mastersizer S particle size analyzer. All samples were tested using purified water as the dispersant and an obscuration of between 12 and 20% was achieved for sample measurement. The results, seen below in Table 6, are mean d90 results of three measurements, the d90 value reflecting the level at which 90% of particles measured are of the specified size or smaller. The following formulation seen below in Table 6 was tested.

**Table 6: Drug A Formulation Comprising SMW Fish GelatinAs Matrix Forming Agent**

| Component (%w/w) | Exp. 12 |
|---|---|
| Purified Water EP/USP | 83.9975 |
| Drug A | 5.0000 |
| Gelatin EP/USP/JP (SMW Fish Gelatin) | 5.5000 |
| Mannitol EP/USP | 5.0000 |
| Aspartame EP/USNF | 0.4000 |
| Sodium Methyl Paraben EP/USNF | 0.0765 |
| Sodium Propyl Paraben EP/USNF | 0.0255 |
| TOTAL | 100% |

The SMW fish gelatin formulation demonstrated consistently low viscosity and particle size at all times tested, as seen below in Table 7.

**Table 7: Viscosity and Particle Size in Drug A Formulation Comprising SMW Fish Gelatin As Matrix Forming Agent**

| | Exp. 12(SMW Fish Gelatin) |
|---|---|
| Viscosity, mPas (0 Hours Stirring) | 10.024 |
| Viscosity, mPas (24 Hours Stirring) | 9.1818 |
| Viscosity, mPas (48 Hours Stirring) | 10.847 |
| Viscosity, mPas (72 Hours Stirring) | 8.4343 |
| Particle Size, mean d90 (0 Hours Stirring) | 14.36 µm |
| Particle Size, mean d90 (24 Hours Stirring) | 13.68 µm |
| Particle Size, mean d90 (48 Hours Stirring) | 13.73 µm |
| Particle Size, mean d90 (72 Hours Stirring) | 13.69µm |

Raman spectroscopy was then used to determine the amount of crystal conversion to the unwanted dihydrate form in the finished units. The results of the Raman spectroscopy testing are collaborated from ratios between peaks to calculate the percentage of dihydrate crystals in the sample. The results, seen below in Table 8, show that little or no crystalline conversion takes place in the fish gelatin matrix system. This is an unexpected finding in at least one respect, as the SMW fish gelatin matrix system displayed a significantly low viscosity, and in general, lower viscosity suspensions are known to facilitate crystalline conversion.

**Table 8: % Dihydrate Crystallization of Drug A Suspensions Seen in Table 7**

| Holding Time (Hours) | 0 | 24 | 48 | 72 |
|---|---|---|---|---|
| Exp. Number | Exp.12 (a) | Exp. 12 (b) | Exp. 12 (c) | Exp. 12 (d) |
| Dihydrate (%) | 0.0 | 0.0 | 0.0 | 0.0 |

Accordingly, the investigations indicated that a synergism between the utilization of SMW fish gelatin as a matrix forming agent and low processing temperatures results in a significant reduction in the rate of crystalline conversion of alprazolam and/or Drug A containing compounds.

Therefore, experiments were expanded to test the efficacy of the SMW fish gelatin and low processing temperature model on yet another drug in the benzodiazepine family, Drug B. Drug B is a benzodiazepine with anti-anxiety, sedative, and anticonvulsant effects. The following formulations were tested, as seen in Table 9.

**Table 9: Drug B Suspensions in Bovine Gelatin and SMW Fish Gelatin as Matrix Forming Agents**

| Component (%w/w) | Exp. 13 | Exp. 14 |
|---|---|---|
| Purified Water EP/USP | 88.40 | 91.90 |
| Drug B | 1.00 | 1.00 |
| SMW Fish Gelatin | 5.50 | |
| Bovine Gelatin | | 4.00 |
| Mannitol EP/USP | 5.00 | 3.00 |
| Sodium Methyl Paraben EP/USNF | 0.078 | 0.018 |
| Sodium Propyl Paraben EP/USNF | 0.025 | 0.057 |
| Sodium Butyl Paraben EP/USNF | 0 | 0.025 |
| TOTALS | 100.00 | 100.00 |

No crystal conversion was seen in Exp. 13 (Drug B in SMW fish gelatin matrix) after 24 hours at 10°C, but some small needles became visible after 48 hours. On the other hand, significant crystal conversion was seen with formulation Exp. 14 (Drug B in bovine gelatin) after 14 hours when held at ambient temperature.

Thus, it has been shown that when fast dispersing dosage forms of drugs from the benzodiazepine class are formulated using SMW fish gelatin as a matrix forming agent, and with processing temperatures kept at a low level, a synergistic effect occurs to minimize the crystalline conversion of the drug in such formulations. Experiments show that this effect cannot be accounted for by either the use of SMW fish gelatin alone, or with the use of low processing temperatures alone.

What is claimed, then, is a process for preparing an oral solid fast dispersing dosage form of a pharmaceutically active substance. The process comprises the steps of forming a suspension, in a continuous phase, of particles of a pharmaceutically active substance in a carrier material that may be standard molecular weight (SMW) fish gelatin. In the process, the temperature of the suspension is reduced to less than about 15°C, and the suspension is held at a temperature of less than about 15°C while forming discrete units of the reduced temperature suspension. The discrete units, often tablets in form, are then processed by means well-known in the art to remove the continuous phase to leave the rapidly dispersing form in the carrier material. In certain embodiments, the continuous phase comprises water.

In various embodiments and pharmaceutical applications, the pharmaceutically active substance may be selected from the group of substances exhibiting crystalline polymorphism. These include, among others, the benzodiazepine family.

As is well-known in the field of fast dispersing dosage forms, the form may have a disintegration/dispersion time of from 1-60 seconds and may be designed for oral administration to release the pharmaceutically active substance rapidly in the oral cavity. The solid dosage form may also contain at least one additional ingredient selected from coloring agents, flavoring agents, excipients, other therapeutic agents and combinations thereof.

The instant invention provides a commercially practical means for the formulation of fast dispersing dosage forms of pharmaceutical agents that display crystal polymorphism, particularly for those agents displaying significant crystal polymorphism when held in suspension for commercially typical periods during formulation. The utilization of a process combining standard molecular weight fish gelatin and low processing temperatures tends to suppress crystalline conversion of such agents.

## Claims

1. A process for preparing an oral, solid fast dispersing dosage form of a pharmaceutically active substance, wherein said substance exhibits polymorphism in an aqueous environment, comprising the steps of:
(a) forming a suspension of particles of said substance in a carrier material in a continuous phase, wherein the carrier material comprises standard molecular weight fish gelatin in which at least 50% of the molecular weight distribution is below 30,000 daltons;
(b) reducing the suspension temperature to less than about 15 °C and maintaining the suspension temperature at less than about 15 °C after step (b) and prior to step (c);
(c) forming discrete units of the suspension at a formation temperature of less than about 15 °C; and
(d) removing the continuous phase to leave the suspension of particles in the carrier material.

2. The process according to claim 1, wherein the continuous phase comprises water.

3. The process according to claim 1, wherein the pharmaceutically active substance is selected from the group consisting of acyclovir, alendronate sodium, amoxicillin, aripiprazole, atorvastatin calcium, carbamazepine, carvedilol, cephalexin, clindamycin, colchicine, donepezil hydrochloride, erythromycin, esomeprazole magnesium, fluoxetine hydrochloride, hydrochlorothiazide, hydrocodone, hyoscyamine sulphate, levofloxacin, levothyroxine sodium, lisinopril, losartan potassium, methotrexate, mirtazapine, mometasone furoate monohydrate, morphine, nystatin, pantoprazole sodium, paroxetine hydrochloride, risedronate sodium, rosiglitazone maleate, tetracycline, theophylline and zithromax.

4. The process according to claim 1, wherein the pharmaceutically active substance is a benzodiazepine drug.

5. The process according to claim 4, wherein the pharmaceutically active substance is alprazolam.

6. The process to claim 1, wherein said fast dispersing dosage form has a disintegration time of from 1 to 60 seconds.

7. The process according to claim 1, wherein said suspension further comprises at least one additional ingredient selected from the group consisting of coloring agents, flavoring agents, excipients, other therapeutic agents and combinations thereof.

8. A process for preparing an oral, solid fast dispersing dosage form of a pharmaceutically active substance as claimed in claim 1, wherein said substance is a benzodiazepine class drug that exhibits crystalline polymorphism in an aqueous environment.

9. The process according to claim 8, wherein the suspension exhibits a fairly consistent viscosity over a period of at least 48 hours.

10. The process according to claim 9, wherein the continuous phase is water.

11. The process according to claim 9 wherein the suspension further comprises at least one additional ingredient selected from the group consisting of coloring agents, flavoring agents, excipients, other therapeutic agents and combinations thereof.

12. The process according to claim 9, wherein the removal of the solvent from the mixture is preferably carried out by freeze drying.

13. A fast dispersing dosage form prepared by the process according to any one of claims 1-12.

14. A fast dispersing dosage form prepared by the process of any one of claims 4-5 for use in a method of treating a central nervous system disorder.

15. The fast dispersing dosage form for use to treat a central nervous system disorder of claim 14, wherein the central nervous system disorder is selected from anxiety disorder, symptomatic dysphasia, panic disorder, convulsive disorder, schizophrenia and bipolar or manic-depressive disorder.

## Patentansprüche

1. Ein Verfahren zur Herstellung einer oralen, festen, schnell dispergierenden Dosierungsform eines pharmazeutischen Wirkstoffs, wobei der Wirkstoff einen Polymorphismus in einer wässrigen Umgebung aufweist, umfassend die Schritte:
(a) Bilden einer Suspension von Partikeln des Wirkstoffs in einem Trägermaterial in einer kontinuierlichen Phase, wobei das Trägermaterial eine Fischgelatine von Standard-Molekulargewicht umfasst, in der mindestens 50 % der Molekulargewichtsverteilung unter 30.000 Dalton liegen;
(b) Absenken der Suspensionstemperatur auf weniger als etwa 15 °C und Aufrechterhalten der Suspensionstemperatur bei weniger als etwa 15 °C nach Schritt (b) und vor Schritt (c);
(c) Bilden diskreter Einheiten der Suspension bei einer Bildungstemperatur von weniger als etwa 15 °C, und
(d) Entfernen der kontinuierlichen Phase, um die Suspension von Partikeln in dem Trägermaterial zu belassen.

2. Verfahren nach Anspruch 1, wobei die kontinuierliche Phase Wasser umfasst.

3. Verfahren nach Anspruch 1, wobei der pharmazeutische Wirkstoff ausgewählt ist aus der Gruppe bestehend aus Acyclovir, Alendronat-Natrium, Amoxicillin, Aripiprazol, Atorvastatin-Calcium, Carbamazepin, Carvedilol, Cephalexin, Clindamycin, Colchicin, Donepezilhydrochlorid, Erythromycin, Esomeprazol-Magnesium, Fluoxetinhydrochlorid, Hydrochlorothiazid, Hydrocodon, Hyoscyaminsulfat, Levofloxacin, Levothyroxin-Natrium, Lisinopril, Losartan-Kalium, Methotrexat, Mirtazapin, Mometasonfuroat-Monohydrat, Morphin, Nystatin, Pantoprazol-Natrium, Paroxetinhydrochlorid, Risedronat-Natrium, Rosiglitazonmaleat, Tetracyclin, Theophyllin und Zithromax.

4. Verfahren nach Anspruch 1, wobei der pharmazeutische Wirkstoff ein Benzodiazepin-Arzneimittel ist.

5. Verfahren nach Anspruch 4, wobei der pharmazeutische Wirkstoff Alprazolam ist.

6. Verfahren nach Anspruch 1, wobei die schnell dispergierende Dosierungsform eine Zerfallszeit von 1 bis 60 Sekunden aufweist.

7. Verfahren nach Anspruch 1, wobei die Suspension mindestens einen weiteren Bestandteil umfasst, ausgewählt aus der Gruppe bestehend aus Farbstoffen, Aromastoffen, Hilfsstoffen, anderen therapeutischen Wirkstoffen und Kombinationen davon.

8. Verfahren zur Herstellung einer oralen, festen, schnell dispergierenden Dosierungsform eines pharmazeutischen Wirkstoffs nach Anspruch 1, wobei der Wirkstoff ein Arzneimittel der Benzodiazepin-Klasse ist, das in einer wässrigen Umgebung einen kristallinen Polymorphismus aufweist.

9. Verfahren nach Anspruch 8, wobei die Suspension über einen Zeitraum von mindestens 48 Stunden eine ziemlich gleichbleibende Viskosität aufweist.

10. Verfahren nach Anspruch 9, wobei die kontinuierliche Phase Wasser ist.

11. Verfahren nach Anspruch 9, wobei die Suspension ferner mindestens einen weiteren Bestandteil umfasst, ausgewählt aus der Gruppe bestehend aus Farbstoffen, Aromastoffen, Hilfsstoffen, anderen therapeutischen Wirkstoffen und Kombinationen davon.

12. Verfahren nach Anspruch 9, wobei die Entfernung des Lösungsmittels aus dem Gemisch vorzugsweise durch Gefriertrocknung erfolgt.

13. Schnelle Dispergierdosierungsform, hergestellt nach dem Verfahren nach einem der Ansprüche 1 bis 12.

14. Eine schnell dispergierende Dosierungsform, hergestellt nach dem Verfahren nach einem der Ansprüche 4 bis 5 zur Verwendung in einem Verfahren zur Behandlung einer Störung des zentralen Nervensystems.

15. Die schnell dispergierende Dosierungsform zur Verwendung zur Behandlung einer Störung des zentralen Nervensystems nach Anspruch 14, wobei die Störung des zentralen Nervensystems aus Angststörung, symptomatischer Dysphasie, Panikstörung, krampfhafter Störung, Schizophrenie und bipolarer oder manisch-depressiver Störung ausgewählt ist.

## Revendications

1. Procédé de préparation d'une forme posologique orale solide à dispersion rapide d'une substance active sur le plan pharmaceutique, ladite substance présentant un polymorphisme dans un environnement aqueux, comprenant les étapes consistant à :
(a) former une suspension de particules de ladite substance dans une matière porteuse dans une phase continue, la matière porteuse comprenant de la gélatine de poisson de masse moléculaire standard dans laquelle au moins 50 % de la distribution de la masse moléculaire est en dessous de 30 000 daltons ;
(b) réduire la température de la suspension à moins d'environ 15 °C et maintenir la température de la suspension à moins d'environ 15 °C après l'étape (b) et avant l'étape (c) ;
(c) former des unités distinctes de la suspension à une température de formation inférieure à environ 15 °C ; et
(d) éliminer la phase continue pour laisser la suspension de particules dans la matière porteuse.

2. Procédé selon la revendication 1, la phase continue comprenant de l'eau.

3. Procédé selon la revendication 1, la substance active sur le plan pharmaceutique étant choisie dans le groupe constitué d'aciclovir, alendronate de sodium, amoxicilline, aripiprazole, atorvastatine calcique, carbamazépine, carvédilol, céfalexine, clindamycine, colchicine, chlorhydrate de donépézil, érythromycine, ésoméprazole magnésium, chlorhydrate de fluoxétine, hydrochlorothiazide, hydrocodone, sulfate d'hyoscyamine, lévofloxacine, lévothyroxine sodique, lisinopril, losartan potassique, méthotrexate, mirtazapine, furoate de mométasone monohydrate, morphine, nystatine, pantoprazole sodique, chlorhydrate de paroxétine, risédronate sodique, maléate de rosiglitazone, tétracycline, théophylline et zithromax.

4. Procédé selon la revendication 1, la substance active sur le plan pharmaceutique étant un médicament benzodiazépine.

5. Procédé selon la revendication 4, la substance active sur le plan pharmaceutique étant l'alprazolam.

6. Procédé selon la revendication 1, ladite forme posologique à dispersion rapide ayant une durée de désintégration de 1 à 60 secondes.

7. Procédé selon la revendication 1, ladite suspension comprenant en outre au moins un ingrédient supplémentaire choisi dans le groupe constitué d'agents colorants, d'agents aromatisants, d'excipients, d'autres agents thérapeutiques et de combinaisons de ceux-ci.

8. Procédé de préparation d'une forme posologique orale solide à dispersion rapide d'une substance active sur le plan pharmaceutique ainsi que revendiqué dans la revendication 1, ladite substance étant un médicament de la catégorie des benzodiazépines qui présente un polymorphisme cristallin dans un environnement aqueux.

9. Procédé selon la revendication 8, la suspension présentant une viscosité assez régulière sur une période d'au moins 48 heures.

10. Procédé selon la revendication 9, la phase continue étant l'eau.

11. Procédé selon la revendication 9, la suspension comprenant en outre au moins un ingrédient supplémentaire choisi dans le groupe constitué d'agents colorants, d'agents aromatisants, d'excipients, d'autres agents thérapeutiques et de combinaisons de ceux-ci.

12. Procédé selon la revendication 9, l'élimination du solvant du mélange étant de préférence effectuée par lyophilisation.

13. Forme posologique à dispersion rapide préparée par le procédé selon l'une quelconque des revendications 1 à 12.

14. Forme posologique à dispersion rapide préparée par le procédé selon l'une quelconque des revendications 4 à 5 destinée à être utilisée dans le traitement d'un trouble du système nerveux central.

15. Forme posologique à dispersion rapide destinée à être utilisée dans le traitement d'un trouble du système nerveux central selon la revendication 14, le trouble du système nerveux central étant choisi parmi un trouble anxieux, une dysphasie symptomatique, un trouble panique, un trouble convulsif, la schizophrénie et un trouble bipolaire ou maniaco-dépressif.
